# EUROPÄISCHE PATENTSCHRIFT

(11) **EP 1 246 785 B1**
(45) Veröffentlichungstag und Bekanntmachung des Hinweises auf die Patenterteilung: **16.06.2004**
(21) Anmeldenummer: 00987467.8
(22) Anmeldetag: 28.12.2000
(51) Int. Cl.: C07C 29/80, C07C 29/141, C07C 31/22

(54) **VERFAHREN ZUR ZERSETZUNG VON BEI DER SYNTHESE MEHRWERTIGER ALKOHOLE GEBILDETER HOCHSIEDENDER NEBENPRODUKTE**
METHOD FOR THE DECOMPOSITION OF HIGH BOILING BY-PRODUCTS PRODUCED IN THE SYNTHESIS OF POLYHYDRIC ALCOHOLS
PROCEDE POUR DECOMPOSER DES SOUS-PRODUITS A POINT D'EBULLITION ELEVE, FORMES LORS DE LA SYNTHESE D'ALCOOLS POLYVALENTS

(30) Priorität: 28.12.1999 DE 19963437
(43) Veröffentlichungstag der Anmeldung: 09.10.2002
(73) Patentinhaber: BASF AKTIENGESELLSCHAFT, 67056 Ludwigshafen (DE)
(72) Erfinder: DERNBACH, Matthias, 69221 Dossenheim (DE); KRATZ, Detlef, 69121 Heidelberg (DE); STAMMER, Achim, 67251 Freinsheim (DE); SCHULZ, Gerhard, 67098 Bad Dürkheim (DE)
(74) Vertreter: Isenbruck, Günter, Dr.
(86) Internationale Anmeldenummer: PCT/EP2000/013308
(87) Internationale Veröffentlichungsnummer: WO 2001/047848

(56) Entgegenhaltungen:
- DD-A- 287 251
- CHEMICAL ABSTRACTS, vol. 78, no. 11, 19. März 1973 (1973-03-19) Columbus, Ohio, US; abstract no. 71411f, Seite 413; XP002165286 & SU 355 141 A (M.P. VYSOTSKII, ET AL.) 16. Oktober 1972 (1972-10-16) in der Anmeldung erwähnt

## Beschreibung

Die vorliegende Erfindung betrifft das Gebiet der industriellen organischen Chemie. Genauer gesagt betrifft die vorliegende Anmeldung ein Verfahren, bei dem mehrwertige Alkohole, die durch Kondensation von Formaldehyd mit höheren Aldehyden und anschließender Hydrierung dargestellt wurden, von bei der Herstellung gebildeten höhersiedenden Substanzen befreit werden.

Aldehyde, die mindestens ein acides Wasserstoffatom in α-Stellung zur Carbonylfunktion aufweisen, bilden bei der Umsetzung mit Formaldehyd in Gegenwart von Basen zunächst methylolierte Aldehyde durch einfache Aldolkondensation. Diese methylolierten Aldehyde dienen dann als Ausgangssubstanz für die Herstellung der erwähnten mehrwertigen Alkohole durch Reduktion der Aldehydgruppe zur Alkoholgruppe.

Dabei wird zwischen verschiedenen Verfahrensvarianten unterschieden, je nach- dem, wie diese Reduktion durchgeführt wird.

Zum einen existiert das anorganische Cannizzaro-Verfahren. Dabei bringt man Formaldehyd mit dem höheren Aldehyd in Gegenwart von stöchiometrischen Mengen einer anorganischen Base, generell NaOH, Ca(OH)₂ oder auch Ba(OH)₂, zur Reaktion. Nach der Kondensation reagiert der methylolierte Aldehyd dann unter dem Einfluß der Base mit weiterem Formaldehyd in einer sogenannten gekreuzten Cannizzaro-Reaktion, wobei durch Disproportionierung der mehrwertige Alkohol und Ameisensäure, in Form seines Salzes, entstehen. Nachteilig ist der Anfall dieses Formiats, das nicht wiederverwendet werden kann und entsorgt werden muß. Dieses belastet die Umwelt, weiterhin geht je Mol gewonnenem Alkohol ein Mol Formaldehyd verloren.

Gemäß einer weit verbreiteten Variante wird im sogenannten organischen Cannizzaro-Verfahren die anorganische Base durch ein tertiäres Amin, im allgemeinen ein Trialkylamin, ersetzt. Auch dieses Amin wird in stöchiometrischen Mengen eingesetzt.

Der Reaktionsverlauf ist wie bei dem anorganischen Cannizzaro-Verfahren, wobei anstelle eines Alkali- oder Erdalkaliformiats das Trialkylammoniumformiat des eingesetzten Amins entsteht. Diese organische Cannizzaro-Reaktion verläuft ohne Bildung von Alkalisalzen, wodurch die Aufarbeitung einfacher wird. Weiterhin kann das Trialkylammoniumformiat weiter aufgearbeitet und das gebundene Amin zurückgewonnen werden, das dann erneut in der Reaktion verwendet wird. Verschiedene Varianten dieser Wiederaufarbeitung von Trialkylammoniumformiaten sind dabei in den Anmeldungen EP-A-289 921, WO 97/17313 und DE-A-198 48 568 beschrieben.

Schließlich ist es auch bekannt, die mehrwertigen Alkohole nach dem sogenannten Hydrierverfahren herzustellen. Dabei wird die Kondensationsreaktion zwischen Formaldehyd und höherem Aldehyd so durchgeführt, daß die Reaktion auf der Stufe des methylolierten Alkanals anhält. Dieses wird erreicht durch Zugabe lediglich katalytischer Mengen eines tertiären Amins, auch hier generell ein Trialkylamin. Die Reduktion der methylolierten Alkanale geschieht durch Hydrierung in an sich bekannter Weise, und man erhält so den gewünschten mehrwertigen Alkohol.

Verschiedene Reaktionsvarianten dieses Hydrierverfahrens finden sich beispielsweise in den Anmeldungen DE-A-25 07 461, DE-A-27 02 582 und DE-A-28 13 201. Ein besonders geeignetes Verfahren wird in der WO 98/28253 offenbart. Hier wird eine vollständige Umsetzung der Edukte zu methylolierten Alkanalen durch ein effektives, vergleichsweise einfaches Auftrennverfahren des primär erhaltenen Reaktionsgemischs und Rückführen der so erhaltenen Fraktionen erzielt. Das erhaltene Alkanal wird dann in an sich bekannter Weise hydriert. In einer ersten Reaktionsstufe wird dabei der Ausgangsaldehyd mit der 2-bis 8-fachen Menge Formaldehyd in Gegenwart von ca. 5 bis 10 Mol-% eines Trialkylamins, vorzugsweise Trimethylamin, umgesetzt. Nach vollendeter Reaktion erfolgt dann die Auftrennung des erhaltenen Gemischs. Dabei wird durch Destillation ein das Alkanal enthaltender Sumpf- und ein nicht umgesetzte Edukte enthaltender Destillatstrom erhalten. Alternativ kann das Rohreaktionsgemisch auch durch Phasentrennung in eine wäßrige und eine organische Phase getrennt werden, wobei die organische Phase nicht umgesetzte Reaktionsprodukte enthält. Destillatstrom bzw. organische Phase werden in die erste Stufe zurückgeführt. So geht nur ein geringer Teil der Ausgangsverbindungen verloren. Der nach Destillation erhaltene Sumpf bzw. die nach Phasentrennung erhaltene wäßrige Phase werden dann einer geeigneten katalytischen bzw. thermischen Behandlung unterworfen, um ein vollständiges Überführen von durch unvollständige Umsetzung oder Eliminierung entstandenen Nebenprodukten in das gewünschte alkylolierte Alkanal zu erreichen. Anschließend wird erneut destilliert und ein Kopfprodukt abgenommen, das in die erste Reaktionsstufe zurückgeführt wird. Der nach dieser letzten Destillation erhaltene Sumpf wird dann hydriert zur Herstellung von mehrwertigem Alkohol.

Die nach dem Hydrierverfahren erhaltenen Rohproduktgemische weisen einen höheren Produktgehalt auf als nach dem Cannizzaro-Verfahren erhaltene Rohgemische. Die Aufarbeitung der nach diesem Verfahren hergestellten Gemische unterscheidet sich daher grundlegend von denjenigen, die nach dem Hydrierverfahren erhalten wurden.

Die Gemeinsamkeiten beider Verfahren liegen lediglich darin, daß der mehrwertige Alkohol destillativ von Komponenten befreit wird, die leichter als dieser flüchtig sind (sogenannte Leichtsieder) bzw. die schwerer als dieser flüchtig sind (sogenannte Hochsieder). Leichtsieder sind dabei insbesondere Wasser, Methanol, bei Verwendung eines Amins als Katalysator das freie Amin und auch Trialkyl-ammoniumformiat.

Bei den Hochsiedern handelt es sich dabei oft um Verbindungen, die Derivate des hergestellten mehrwertigen Alkohols sind und aus diesem durch Reaktion mit beispielsweise Formaldehyd, Methanol oder auch einem weiteren Molekül des hergestellten Alkohols entstanden sind. Typische höhersiedende Nebenkomponenten sind beispielsweise, für den Fall der Synthese des dreiwertigen Alkohols Trimethylolpropan (TMP) C₂H₅C(CH₂OH)₃ aus Formaldehyd und n-Butyraldehyd in Gegenwart von katalytischen Mengen Trialkylamin, die nachfolgend genannten Verbindungen. Das ist zum einen das sogenannten Di-TMP [C₂H₅C(CH₂OH)₂CH₂]₂O, das lineare Bis-TMP-Formal [C₂H₅C(CH₂OH)₂CH₂O]CH₂, das cyclische TMP-Formal 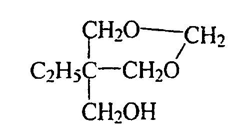 das Kondensationsprodukt von TMP, Formaldehyd und Methanol (TMP-FA-MeOH) das Acetal von Dimethylolbutanal mit Trimethylolpropan (DMB-TMP-Acetal) sowie TMP-Formiate, also Ameisensäuremonoester von Trimethylolpropan.

Es ist offensichtlich, daß die Bildung dieser TMP-Einheiten enthaltenden Hochsieder unerwünscht ist, da sie die Ausbeute an gewünschtem Produkt deutlich senken. Jedoch läßt sich die Bildung dieser Hochsieder nie ganz unterdrücken, auch bei vorsichtiger Reaktionsführung. Um die Ausbeute zu erhöhen, ist es wünschenswert, diese Hochsieder zu zersetzen. Dabei werden in der Literatur verschiedene Verfahren offenbart, um dieses zu erreichen.

Die Patentschrift DD-P-287 251 beschreibt die Zersetzung von Hochsiedern, insbesondere Bis-TMP-Formal, in Lösungen, die bei der Herstellung von TMP nach dem anorganischen Cannizzaro-Verfahren nach destillativem Abtrennen des erhaltenen TMP gewonnen wurden. Das so erhaltene Sumpfprodukt wird dann mit 1 bis 10 Gew.-% einer starken oder mittelstarken Säure versetzt, beispielsweise Schwefelsäure oder Phosphorsäure, und anschließend 6 Minuten bis 10 Stunden auf Temperaturen von 80 bis 180°C erhitzt. Es resultiert eine Spaltung einiger von TMP abgeleiteter Hochsieder. Beispielsweise läßt sich durch Zugabe von 5 Gew.-% konzentrierter Schwefelsäure zu einem Hochsieder-Rohgemisch und Erhitzen auf 130°C für 12 Minuten mit anschließender Destillation zum Gewinnen von TMP ein vollständiges Zersetzen von Bis-TMP-Formal und somit eine Ausbeutesteigerung an TMP erreichen. Es wird dabei jedoch auch in erheblichen Mengen vorher nicht vorhandenes cyclisches TMP-Formal gebildet, was nicht erwünscht ist.

In der SU 355141, dessen Offenbarung praktisch mit der des Artikels "The Soviet Chemical Industry" 1977, 9:8, Seite 599 bis 600 identisch ist, wird ein Verfahren offenbart, bei dem die Leichtsiederfraktion von nach dem anorganischen Cannizzaro-Verfahren erhaltenem TMP aufgearbeitet wird, indem 1 bis 8 Gew.-% Schwefelsäure zugegeben werden und man in die Lösung überhitzten Wasserdampf einer Temperatur von 180 bis 200°C einleitet, bei einer Sumpftemperatur von 170 bis 180°C. Von diesem Sumpf wird anschließend TMP abdestilliert. Es läßt sich so eine Gesamtausbeutesteigerung an TMP durch Hydrolyse von cyclischem TMP-Formal sowie TMP-Formiaten erreichen, wobei eine Ausbeute an TMP in dem Zersetzungsschritt mit 18 bis 20 % angegeben wird. Es entsteht jedoch auch in 53 bis 55 % Ausbeute ein unerwünschtes, höhersiedendes Produkt.

Schließlich ist auch in der US 3,259,662 ein Verfahren offenbart, bei dem eine nach dem anorganischen Cannizzaro-Verfahren erhaltene TMP-Rohlösung unter Zusatz von Säure destilliert wird. Die Säure wird dabei während der Destillation der leichter siedenden Komponenten, nach dem Entfernen von Formaldehyd, zugegeben. Anschließend wird das TMP von den Hochsiedern abdestilliert, wobei im Sumpf ein alkalischer pH-Wert eingestellt wurde. Damit läßt sich ein geruchloses TMP gewinnen.

Die oben beschriebenen Verfahren eignen sich jedoch nicht zur Aufarbeitung von nach dem Hydrierverfahren erhaltenem TMP oder anderen mehrwertigen Alkoholen. Das aufzuarbeitende Reaktionsgemisch unterscheidet sich deutlich von Reaktionsausträgen des Cannizzaro-Verfahrens. Es enthält keine Alkali- oder Erdalkaliionen und nur geringe Mengen an tertiärem Amin bzw. dem entsprechenden Trialkylammoniumformiat. Durch einfaches Entwässern des nach Hydrieren erhaltenen Alkohols läßt sich ein Rohalkoholgemisch erhalten, das einen Produktgehalt von 80 bis 85 % aufweist. Im Gegensatz zu Rohgemischen, die nach dem Cannizzaro-Verfahren (organisch oder anorganisch) erhalten wurden, läßt sich reines TMP aus den entwässerten Rohlösungen durch destillative Schritte erhalten. Es müssen geringe Mengen an Verunreinigungen abgetrennt werden. Der Zusatz großer Mengen an Säure, zusätzliches Einleiten von etwa Wasserdampf und zusätzliche Abtrennschritte sind nicht erwünscht, da eine Ausbeuteverbesserung zwar durch Zersetzung der Adukte von TMP sehr erwünscht ist, die Menge dieser Adukte aber beim Hydrierverfahren geringer ist als beim Cannizzaro-Verfahren und deshalb keinen hohen Aufwand rechtfertigt.

Die Aufgabe der vorliegenden Erfindung ist daher, ein Verfahren bereitzustellen, mit dem die Ausbeute an mehrwertigem Alkohol, der nach dem Hydrierverfahren hergestellt wurde, verbessert wird. Dieses Verfahren soll effektiv und nicht aufwendig sein, gleichzeitig jedoch die Ausbeute an mehrwertigem Alkohol so verbessern, daß sich ein Einsatz beim Hydrierverfahren lohnt.

Diese Aufgabe wird gelöst durch ein Verfahren zur Ausbeuteerhöhung bei der Herstellung von aus methylolierten Alkanalen durch Hydrierung erhaltenen mehrwertigen Alkoholen durch Zersetzen von Derivaten dieser Alkohole. wobei einem diese Derivate enthaltenden Gemisch 5 ppm bis 1 Gew.-%, vorzugsweise 100 bis 1000 ppm, einer geeigneten Säure zugesetzt werden, das Gemisch auf Temperaturen von 150 bis 280°C erhitzt und anschließend der mehrwertige Alkohol durch Destillation abgetrennt wird.

Es wurde überraschenderweise gefunden, daß durch den Zusatz von geringen Mengen an Säure zu der Hochsieder enthaltenden Fraktion, die bei der Herstellung von mehrwertigen Alkoholen nach dem Hydrierverfahren anfällt, sowie dem Erhitzen auf hohe Temperaturen bis zu 300°C eine deutliche Zersetzung der von dem jeweiligen mehrwertigen Alkohol abgeleiteten, hochsiedenden Komponenten erreicht werden kann. Durch dieses einfache Verfahren ergibt sich eine Ausbeutesteigerung, die bis zu mehreren Prozent betragen kann. Nach dem erfindungsgemäßen Verfahren wird die Synthese der mehrwertigen Alkohole. also die Kondensationsreaktion zwischen Formaldehyd und höherem Aldehyd in Anwesenheit katalytischer Mengen tertiären Amins, gefolgt von einer katalytischen Hydrierung dieses Gemischs, wie in der Literatur beschrieben durchgeführt. Beispiele verschiedener Verfahrensvarianten finden sich dabei in den Anmeldungen DE-A-25 07 461, DE-A-27 02 582 und DE-A-28 13 201, die bereits oben zitiert wurden. Das erfindungsgemäße Verfahren eignet sich besonders zur Hochsiederentfemung bei Mischungen, die nach dem in der WO 98/28253 beschriebenen Verfahren hergestellt wurden. Eine kurze Beschreibung dieses Verfahrens findet sich weiter oben. Die Aufarbeitung erfolgt dann in üblicher Weise, wie in der Literatur beschrieben, generell durch Abtrennen von Wasser und anschließender Destillation.

Im Rahmen der vorliegenden Erfindung wurden gute Ergebnisse mit Rohalkoholgemischen erzielt, die zuvor entwässert wurden, was mit den üblichen Verfahren erfolgen kann. Dabei sind Wassergehalte von ≤ 5 Gew.-% wünschenswert, besser sind Wassergehalte von ≤ 0,5 Gew.-%.

Die Menge an Säure, die nach der vorliegenden Erfindung zur Zersetzung der Hochsieder dem entwässerten Gemisch beigegeben wird, ist deutlich geringer als bei den im Stand der Technik vorhandenen Verfahren, die sich alle auf das Cannizzaro-Verfahren beziehen. Erfindungsgemäß werden 5 ppm bis 1 Gew.-% Säure benutzt, die bevorzugten Mengen liegen bei 100 bis 1000 ppm. Häufig wurde beobachtet, daß bei Zugabe von Säuremengen, die oberhalb des hier angegebenen Bereichs liegen, eine Polymerisation des Gemischs beim Erhitzen oder bei der dem Abtrennen von gebildetem Alkohol dienenden Destillation eintritt.

Als Säuren können erfindungsgemäß übliche starke oder mittelstarke Säuren verwendet werden. Diese sind einem Fachmann bekannt. Beispiele sind Schwefelsäure, schweflige Säure. Halogenwasserstoffsäuren wie HCl, in gasförmiger oder auch wäßriger Form, Phosphorsäure. Sulfonsäuren wie Aryl- und Alkylsulfonsäuren, saure Ionenaustauscher, phosphorige Säure, Borsäure, Alkansäuren und Kohlensäure. Es ist bevorzugt, Phosphorsäure zu verwenden.

Die erfindungsgemäße Hochsiederspaltung findet statt bei Temperaturen von 150 bis 280°C, vorzugsweise 180 bis 250°C. Dabei werden auf den TMP-Zulauf bezogene Verweilzeiten gewählt, die von 0,1 bis 20 Stunden, vorzugsweise 0,5 bis 5 Stunden reichen.

Das erfindungsgemäße Verfahren ist nicht ausgeprägt druckabhängig. Die Zersetzung kann im Vakuum, unter Normaldruck oder auch unter Anlegen eines äußeren Drucks durchgeführt werden. Es ist bevorzugt, wenn die Reaktion unterhalb Atmosphärendruck bei 1 - 950 mbar, vorzugsweise 10 - 100 mbar, meist bevorzugt 10 - 50 mbar, durchgeführt wird. Dabei kann ohne Inertgasatmosphäre oder mit einer solchen, wie beispielsweise einer Argon- oder Stickstoffatmosphäre, gearbeitet werden.

Die vorstehend aufgeführten Reaktionsbedingungen, die zum Zersetzen der Hochsieder nach der vorliegenden Erfindung benutzt werden, können dabei zu verschiedenen Zeitpunkten und während unterschiedlicher Verfahrensstadien während der Aufarbeitung des entwässerten Rohalkoholgemischs verwendet werden. Zum einen ist es beispielsweise möglich, beim destillativen Abtrennen des Produktalkohols von den Hochsiedern einfach dem Sumpf die erforderliche Säuremenge zuzugeben und ansonsten bei der Destillation die Bedingungen einzuhalten, die zur Spaltung der Hochsieder notwendig sind und die oben dargelegt wurden. Die Menge an Produktalkohol in dem abdestillierenden Gemisch läßt sich so erhöhen.

Es ist auch möglich, die Hochsiederfraktion durch geeignete Maßnahmen von dem Produkt und den Leichtsiedern abzutrennen, beispielsweise durch Destillation. In einer separaten Stufe wird dann mit der Hochsiederfraktion das erfindungsgemäße Verfahren durchgeführt und der durch Zersetzen der Hochsieder erhaltene Produktalkohol abdestilliert. Dieser Produktalkohol kann dann als solcher isoliert werden, gegebenenfalls nach weiterer Reinigung durch Destillation. Es ist aber auch möglich, das Destillat in eine der vorhergehenden Destillationsstufen, in der der Produktalkohol in reiner Form durch Destillation erhalten wird, zurückzuführen. Die Zersetzungsreaktion kann beispielsweise in einem Rohreaktor oder einem Rührkessel stattfinden.

Die erfindungsgemäße Hochsiederzersetzung läßt sich besonders gut durchführen in Kombination mit dem in der deutschen Anmeldung mit dem Titel "Verfahren zur Reinigung von durch Hydrierung erhaltenem Trimethylolpropan durch kontinuierliche Destillation", Aktenzeichen 199 63 435.1 (Anmelderin: BASF AG) beschriebenen Verfahren. Dabei wird durch Hydrierung von 2,2-Dimethylolbutanal stammendes Trimethylolpropan (TMP) destillativ aufgearbeitet, wobei in der ersten Stufe von dem nach Hydrierung erhaltenen Gemisch Wasser und andere Leichtsieder wie Methanol, Trialkylamin und auch Trialkylammoniumformiat durch Destillation abgetrennt werden. Von dem so gewonnenen Gemisch enthaltend TMP, Hochsieder und einen Teil der Leichtsieder werden, eventuell nach einer weiteren Behandlung zur Umwandlung und Abtrennung von Nebenprodukten, TMP und Leichtsieder destillativ von den Hochsiedern getrennt und dann aufgearbeitet. Die Hochsiederfraktion kann verworfen oder teilweise in die Reaktion zurückgeschleust werden. Die Offenbarung der oben genannten Anmeldung ist durch Referenz in die vorliegende Anmeldung einbezogen.

Wird nun bei dem in dieser zitierten Anmeldung offenbarten Verfahren während der Abtrennung des TMP und der anderen Leichtsieder von den Hochsiedern dem Sumpf Säure in den erfindungsgemäßen Konzentrationen zugesetzt und bei der Destillation die erfindungsgemäßen Bedingungen eingehalten, so läßt sich damit eine Ausbeutesteigerung erreichen. Es ist aber auch möglich, den Hochsiedersumpf nach der Destillation separat nach dem erfindungsgemäßen Verfahren zu behandeln und das erhaltene TMP abzudestillieren.

In einer weiteren bevorzugten Ausführungsform der vorliegenden Erfindung wird dem Rohaustrag nach Entwässern ein wasserfreies Dialkylamin zugefügt, um Formiate des mehrwertigen Alkohols zu zersetzen, und zwar zum entsprechenden Alkohol und Formamid. Ein solches Verfahren ist offenbart in der deutschen Patentanmeldung mit dem Titel "Verfahren zur Umwandlung von bei der Trimethylolalkan-Herstellung anfallenden Trimethylolalkanformiat", Aktenzeichen 199 63 444.0 (Anmelderin: BASF AG). Auch dieses Verfahren ist ein integraler Bestandteil der vorliegenden Anmeldung und durch Referenz in diese einbezogen. Nach der Zersetzung der Formiate erfolgt dann die erfindungsgemäße Aufarbeitung unter Zusatz von Säure.

Das Verfahren ist auf alle mehrwertigen Alkohole anwendbar, die durch Kondensation von Formaldehyd mit höheren Aldehyden unter Zugabe katalytischer Mengen Trialkylamin und nachfolgender Hydrierung hergestellt werden können. Geeignete höhere Aldehyde sind praktisch alle Alkanale mit einem aciden Wasserstoffatom in α-Stellung zur Carbonylgruppe. Es können aliphatische Aldehyde mit 2 bis 24 C-Atomen als Ausgangsmaterialien verwendet werden, die geradkettig oder verzweigt sein oder auch alicyclische Gruppen enthalten können. Ebenso sind araliphatische Aldehyde als Ausgangsstoffe geeignet, vorausgesetzt daß sie eine Methylengruppen in α-Stellung zur Carbonylgruppe enthalten. Im allgemeinen werden Aralkylaldehyde mit 8 bis 24 C-Atomen, vorzugsweise mit 8 bis 12 C-Atomen, als Ausgangsmaterialien verwendet, beispielsweise Phenylacetaldehyd. Bevorzugt werden aliphatische Aldehyde mit 2 bis 12 C-Atomen, beispielsweise 3-Ethyl-, 3-n-Propyl-, 3-Isopropyl-, 3-n-Butyl-, 3-Isobutyl-, 3-sek.-Butyl-, 3-tert.-Butyl-butanal sowie entsprechende n-pentanale, -n-hexanale, -n-heptanale; 4-Ethyl-, 4-n-Propyl-, 4-Isopropyl-, 4-n-Butyl-, 4-Isobutyl-, 4-sek.-Butyl-, 4-tert.-Butyl-pentanale, -n-hexanale, -n-heptanale; 5-Ethyl-, 5-n-Propyl-, 5-Isopropyl-, 5-n-Butyl-, 5-Isobutyl-, 5-sek.-Butyl-, 5-tert.-Butyl-n-hexanale, -n-heptanale; 3-Methyl-hexanal, 3-Methyl-heptanal; 4-Methyl-pentanal, 4-Methyl-heptanal, 5-Methyl-hexanal, 5-Methylheptanal; 3,3,5-Trimethyl-n-pentyl-, 3,3-Diethyl-pen-tyl-, 4,4-Diethylpentyl-, 3,3-Dimethyl-n-butyl-, 3,3-Dimethyl-n-pentyl-, 5,5-Dimethylheptyl-, 3,3-Dimethylheptyl-, 3,3,4-Trimethylpentyl-, 3,4-Dimethylheptyl-, 3,5-Dimethylheptyl-, 4,4-Dimethylheptyl-, 3,3-Diethylhexyl-, 4,4-Dimethylhexyl-, 4,5-Dimethylhexyl-, 3,4-Dimethylhexyl-, 3,5-Dimethylhexyl-, 3,3-Dimethylhexyl-, 3,4-Diethylhexyl-, 3-Methyl-4-ethylpentyl-, 3-Methyl-4-ethylhexyl-, 3,3,4-Trimethylpentyl-, 3,4,4-Trimethylpentyl-, 3,3,4-Trimethylhexyl-, 3,4,4-Trimethylhexyl-, 3,3,4,4,-Tetramethylpentylaldehyd; insbesondere C₂ bis C₁₂-n-Alkanale.

Besonders bevorzugte mehrwertige Alkohole im Rahmen der vorliegenden Erfindung sind Trimethylolethan, Trimethylolpropan, Trimethylolbutan, Neopentylglykol und Pentaerythrit. Der meist bevorzugte Alkohol ist Trimethylolpropan.

Die Erfindung wird jetzt in den nachfolgenden Beispielen erläutet.

### Beispiel 1

### Roh-TMP wurde wie folgt dargestellt:

Eine Apparatur bestehend aus zwei beheizbaren, durch Überlaufrohre miteinander verbundenen Rührkesseln mit einem Fassungsvermögen von insgesamt 72 l wurde mit frischer, wäßriger Formaldehydlösung (4300 g/h in Form der 40 %igen wäßrigen Lösung und n-Butyraldehyd (1800 g/h) und mit frischem Trimethylamin als Katalysator (130 g/h) in Form der 45 %igen wäßrigen Lösung kontinuierlich beschickt. Der Reaktoren wurden dabei auf 40°C temperiert.

Der Austrag wurde direkt in den oberen Teil eines Fallfilmverdampfers mit aufgesetzter Kolonne (11 bar Heizdampf) geleitet und dort bei normalem Druck destillativ in ein leichtsiedendes Kopfprodukt, im wesentlichen enthaltend n-Butyraldehyd, Ethylacrolein, Formaldehyd, Wasser und Trimethylamin, und ein hochsiedendes Sumpfprodukt aufgetrennt.

Das Kopfprodukt wurde kontinuierlich kondensiert und in die oben beschriebenen Reaktoren zurückgeführt.

Das hochsiedende Sumpfprodukt aus dem Verdampfer (ca. 33,5 kg/h) wurde kontinuierlich mit frischem Trimethylamin-Katalysator (50 g/h, in Form der 45 %igen wäßrigen Lösung) versetzt und in einen beheizbaren, mit Füllkörpern versehenen Rohrreaktor mit einem Leervolumen von 12 l geführt. Der Reaktor war dabei auf 40°C temperiert.

Der Austrag des Nachreaktors wurde kontinuierlich in den oberen Teil einer weiteren Destillationseinrichtung, der Formaldehydabtrennung (11 bar Heizdampf), gegeben und dort destillativ in ein leichtsiedendes Kopfprodukt, im wesentlichen enthaltend Ethylacrolein, Formaldehyd, Wasser und Trimethylamin, und ein hochsiedendes Sumpfprodukt aufgetrennt. Das leichtsiedende Kopfprodukt (27 kg/h) wurde kontinuierlich kondensiert und in den ersten Rührkessel zurückgeleitet, wohingegen das hochsiedende Sumpfprodukt gesammelt wurde.

Das so erhaltene Sumpfprodukt enthielt neben Wasser im wesentlichen Dimethylolbutyraldehyd, Formaldehyd und Spuren Monomethylolbutyraldehyd. Es wurde dann einer kontinuierlichen Hydrierung unterworfen. Dazu wurde die Reaktionslösung bei 90 bar und 115°C in einem Hauptreaktor in Kreislauf-/Rieselfahrweise und einem nachgeschalteten Nachreaktor in Kreislauffahrweise hydriert. Der Katalysator wurde analog D der DE 198 09 418 hergestellt. Er enthielt 24% CuO, 20% Cu und 46% TiO₂. Die verwendete Apparatur bestand aus einem 10 m langen beheizten Nachreaktor (Innendurchmesser: 25 mm). Der Kreislaufdurchsatz betrug 25 l/h Flüssigkeit, der Reaktorzulauf wurde auf 4 kg/h eingestellt. Dementsprechend wurden 4 kg/h Hydrieraustrag erhalten.

Das nach der Hydrierung erhaltene Gemisch wurde anschließend gemäß der deutschen Anmeldung, mit dem Titel "Verfahren zur Reinigung von durch Hydrierung erhaltenem Trimethylolpropan durch kontinuierliche Destillation", Aktenzeichen 199 63 435.1 (Anmelderin: BASF AG) von Wasser und Trimethylamin befreit. Das Rohgemisch wurde zunächst der Leichtsiederkolonne zugeführt, wobei die Entwässerung bei 400 mbar und 180°C durchgeführt wurde. Der Zulauf erfolgte in der Mitte der Kolonne. Das Rücklaufverhältnis wurde zu 0,3 gewählt. Es wurde ein Sumpfaustrag (1,1 kg/h) erhalten, der die folgende, gaschromatographisch ermittelte Zusammensetzung aufwies:

**Tabelle 1**

| **2-Ethyl- 1,3- propandiol** | **cyclisches TMP- formal** | **TMP** | **TMP- monoformiat** | **TMP-FA- MeOH** | **DMB- TMP- Acetat** |
|---|---|---|---|---|---|
| 0.8 | 0,6 | 82,6 | 7,7 | 1,7 | 2,2 |

Von diesem Sumpfaustrag wurden anschließend in einer Packungskolonne bei 20 mbar Druck 1.15 kg/h TMP über Kopf abdestilliert, bei einer Kopftemperatur von 184°C. Die Sumpftemperatur betrug 230°C. 66g/h des Kolonnensumpfs wurden in regelmäßigen Abständen ausgeschleust. Während der Destillation wurde alle 3 Tage 1 ml 85 %-ige Phosphorsäure in den Kolonnensumpf gegeben. So wurde eine Säurekonzentration im Sumpf von ca. 90 bis 530 ppm aufrechterhalten. Es wurden über Kopf 1,07 kg/h Roh-TMP abdestilliert. Die Zusammensetzung dieses Produkts war nach gaschromatographischer Analyse (vorherige Silylierung des TMP mit Methylsilyltrifluoracetamid) wie folgt:

**Tabelle 2**

| **2-Ethyl-1,3- propandiol** | **cyclisches TMP-formal** | **TMP** | **TMP- monoformiat** | **TMP-FA- MeOH** |
|---|---|---|---|---|
| 1,4 | 1,2 | 90,2 | 4,1 | 0,8 |

Somit konnten insgesamt 965 g/h TMP über Kopf abdestilliert werden.

### Vergleichsbeispiel 1

Die Destillation wurde durchgeführt wie in Beispiel 1 beschrieben, wobei jedoch keine Phosphorsäure zugegeben und 1,02 kg/h TMP entnommen wurde. Das erhaltene Produkt wies nach gaschromatographischer Analyse die folgende Zusammensetzung auf:

**Tabelle 3**

| **2-Ethyl-1,3- propandiol** | **cyclisches TMP-formal** | **TMP** | **TMP- monoformiat** | **TMP-FA- MeOH** |
|---|---|---|---|---|
| 0,7 | 0,9 | 89,8 | 5,5 | 1,0 |

Somit konnten insgesamt 916 g/h TMP über Kopf abdestilliert werden.

### Beispiel 2

1000 g eines TMP-Rohaustrags, der wie in Beispiel 1 beschrieben hergestellt und entwässert wurde, wurde bei 2 mbar über eine Füllkörperkolonne der Höhe 20 cm und des Durchmessers 2,9 cm diskontinuierlich rektifiziert. Vor Destillationsbeginn wurden 250 ppm Phosphorsäure in Form einer 85 %-igen wäßrigen Lösung zugegeben. Die Anfangstemperatur des Sumpfs betrug 180°C und wurde allmählich auf 235°C erhöht. Es wurden die drei folgenden Fraktionen erhalten:
18 g Leichtsieder (Kopftemperatur 60 bis 145°C)
915 g Hauptlauf (Kopftemperatur 148 bis 165°C)
40 g Rückstand

Die gaschromatographische Analyse des Hauptlaufs zeigte, daß insgesamt 828 g TMP überdestilliert werden konnten.

### Beispiel 3

Es wurde wie in Beispiel 2 beschrieben gearbeitet, jedoch wurden 1000 ppm H₃PO₄ in Form einer 85 %-igen wäßrigen Lösung zugegeben. Es wurden wiederum 3 Fraktionen erhalten, die bei den gleichen Temperaturen wie in Beispiel 2 entnommen wurden. Es konnten
17 g Leichtsieder
932 g Hauptlauf und
20 g Rückstand
erhalten werden. Die gaschromatographische Analyse des Hauptlaufs zeigte, daß insgesamt 815 g TMP überdestilliert werden konnten.

### Vergleichsbeispiel 2

Es wurde verfahren wie in Beispiel 2 beschrieben, wobei jedoch keine Phosphorsäure zugegeben wurde. Es wurden wiederum 3 Fraktionen erhalten, die bei den gleichen Temperaturen wie in Beispiel 2 entnommen wurden. Es konnten
31 g Leichtsieder
860 g Hauptlauf und
100 g Rückstand
erhalten werden. Die gaschromatographische Analyse des Hauptlaufs zeigte, daß 797 g TMP überdestilliert werden konnten.

### Beispiel 4

1000g nach Beispiel 1 hergestellter entwässerter TMP-Rohaustrag wurden mit 41 g Diethylamin versetzt und bei 55°C 1h gerührt. Das überschüssige Diethylamin wurde anschließend destillativ entfernt und zu der verbleibenden Mischung wur- den 250 ppm H₃PO₄ (85 % wässrige Lösung) hinzugefügt.

Die Mischung wurde anschließend analog Beispiel 2 destilliert. Es wurden wiederum 3 Fraktionen erhalten, die bei den gleichen Temperaturen wie in Beispiel 2 entnommen wurden:
57 g Leichtsieder
890 g Hauptlauf
43 Rückstand

Die GC-Analyse des Hauptlaufs ergab, daß insgesamt 848 g TMP im Hauptlauf überdestilliert werden konnten.

### Beispiel 5

Es wurden 400 g des gemäß Beispiel 1 aus dem Sumpf ausgeschleusten Hochsiederstroms diskontinuierlich rektifiziert. Dabei wurde eine Füllkörperkolonne der Höhe 20 cm und des Durchmessers 2,9 cm benutzt, der Druck betrug 2 mbar. Dem Sumpf wurden 125 ppm Phosphorsäure in Form einer 85 %-igen wäßrigen Lösung zugegeben. Die Sumpftemperatur betrug anfänglich 180°C und wurde bis zu einer Temperatur von 235°C gesteigert. 152 g eines bei einer Kopftemperatur von 160 bis 190°C entnommenen Destillats wurden erhalten, gleichzeitig ergab sich 240 g Rückstand. Die gaschromatographische Analyse des Destillats ergab, daß insgesamt 104 g TMP überdestilliert werden konnten.

### Beispiel 6

Es wurde wie in Beispiel 5 beschrieben verfahren, wobei die zugesetzte Menge an 85 %iger Phosphorsäure 625 ppm betrug. Es wurden 206 g Destillat, entnommen bei einer Kopftemperatur von 160 bis 170°C, erhalten, ebenso wie 186 g Rückstand. Die gaschromatographische Analyse des Destillats zeigte, daß insgesamt 158 g TMP überdestilliert werden konnten. Es verblieb ein glasartig erstarrter Rückstand.

### Vergleichsbeispiel 3

Es wurde verfahren, wie dies in Beispiel 5 beschrieben ist, jedoch ohne Zusatz von Phosphorsäure. 73 g Destillat wurden bei einer Kopftemperatur von 150 bis 195°C entnommen, der erhaltene Rückstand fiel in einer Menge von 320 g an. Die gaschromatographische Analyse des Destillats zeigte, daß insgesamt 55 g TMP überdestilliert werden konnten.

## Patentansprüche

1. Verfahren zur Ausbeuteerhöhung bei der Herstellung von aus methylolierten Alkanalen durch Hydrierung erhaltenen mehrwertigen Alkoholen durch Zersetzen von Derivaten dieser Alkohole, wobei einem diese Derivate enthaltenden Gemisch 5 ppm bis 1 Gew.-% einer geeigneten Säure zugesetzt, das Gemisch auf Temperaturen von 150 bis 280°C erhitzt und anschließend der mehrwertige Alkohol durch Destillation abgetrennt wird.

2. Verfahren nach Anspruch 1, **dadurch gekennzeichnet, daß** die Temperatur von 180 bis 250°C, beträgt.

3. Verfahren nach Anspruch 1 oder 2, **dadurch gekennzeichnet, dass** 100 bis 1000 ppm einer geeignten Säure zugesetzt werden.

4. Verfahren nach einem der Ansprüche 1 bis 3, **dadurch gekennzeichnet, daß** die Reaktion unter erhöhtem Druck, bei Atmosphärendruck oder bei vermindertem Druck durchgeführt wird.

5. Verfahren nach einem der Ansprüche 1 bis 4, **dadurch gekennzeichnet, daß** das die Derivate der mehrwertigen Alkohole enthaltende Gemisch nur geringe Mengen Wasser enthält, vorzugsweise ≤ 5 Gew.-% Wasser, meistbevorzugt ≤ 0,5 Gew.-% Wasser.

6. Verfahren nach einem der Ansprüche 1 bis 5, **dadurch gekennzeichnet, daß** die Verweilzeiten bezogen auf den Alkohol-Zulauf 0,1 bis 20 Stunden, vorzugsweise 0,5 bis 5 Stunden, betragen.

7. Verfahren nach einem der Ansprüche 1 bis 6, **dadurch gekennzeichnet, daß** eine starke oder mittelstarke Säure, vorzugsweise Phosphorsäure, verwendet wird.

8. Verfahren nach einem der Ansprüche 1 bis 7, **dadurch gekennzeichnet, daß** die mehrwertigen Alkohole ausgewählt sind aus der Gruppe bestehend aus Trimethylolethan, Trimethylolpropan, Trimethylolbutan, Neopentylglykol und Pentaerythrit, meist bevorzugt Trimethylolpropan.

9. Verfahren nach einem der Ansprüche 1 bis 8, **dadurch gekennzeichnet, daß** vor der Säurezugabe dem Gemisch ein sekundäres Amin zugegeben wird.

10. Verfahren nach einem der Ansprüche 1 bis 9, **dadurch gekennzeichnet, daß** das Verfahren bei Gemischen durchgeführt wird, aus denen der mehrwertige Alkohol noch nicht destillativ entfernt wurde.

11. Verfahren nach einem der Ansprüche 1 bis 9, **dadurch gekennzeichnet, daß** das Verfahren bei Gemischen durchgeführt wird, aus denen der mehrwertige Alkohol bereits destillativ entfernt wurde.

## Claims

1. A process for increasing the yield in the preparation of polyhydric alcohols obtained from methylolated alkanals by hydrogenation, wherein derivatives of these alcohols are decomposed by adding 5 ppm to 1% by weight of a suitable acid to a mixture containing these derivatives, heating the mixture to temperatures of 150 to 280°C and then separating off the polyhydric alcohol by distillation.

2. A process as claimed in claim 1 wherein the temperature is from 180 to 250°C.

3. A process as claimed in claim 1 or 2 wherein 100 to 1000 ppm of a suitable acid are added.

4. A process as claimed in any of claims 1 to 3 wherein the reaction is carried out under superatmospheric pressure, under atmospheric pressure or under reduced pressure.

5. A process as claimed in any of claims 1 to 4 wherein the mixture containing the derivatives of the polyhydric alcohols only contains small amounts of water, preferably ≤5% by weight of water and most preferably ≤0.5% by weight of water.

6. A process as claimed in any of claims 1 to 5 wherein the residence times in respect of the alcohol feed are 0.1 to 20 hours, preferably 0.5 to 5 hours.

7. A process as claimed in any of claims 1 to 6 wherein a strong or moderately strong acid, preferably phosphoric acid, is used.

8. A process as claimed in any of claims 1 to 7 wherein the polyhydric alcohols are selected from the group consisting of trimethylolethane, trimethylolpropane, trimethylolbutane, neopentyl glycol and pentaerythritol, trimethylolpropane being particularly preferred.

9. A process as claimed in any of claims 1 to 8 wherein a secondary amine is added to the mixture before the acid is added.

10. A process as claimed in any of claims 1 to 9 which is carried out on mixtures from which the polyhydric alcohol has not yet been removed by distillation.

11. A process as claimed in any of claims 1 to 9 which is carried out on mixtures from which the polyhydric alcohol has already been removed by distillation.

## Revendications

1. Procédé destiné à augmenter le rendement lors de la préparation d'alcools polyvalents obtenus par hydrogénation à partir d'alcanals méthylolés en décomposant les dérivés de ces alcools, 5 ppm à 1 % en poids d'un acide approprié étant ajouté à un mélange contenant ces dérivés, le mélange étant chauffé à des températures allant de 150 à 280°C et l'alcool polyvalent étant ensuite séparé par distillation.

2. Procédé selon la revendication 1, **caractérisé en ce que** la température va de 180 à 250°C.

3. Procédé selon la revendication 1 ou 2, **caractérisé en ce que** 100 à 1000 ppm d'un acide approprié sont ajoutés.

4. Procédé selon l'une des revendications 1 à 3, **caractérisé en ce que** la réaction s'effectue sous pression élevée, sous pression atmosphérique ou sous pression réduite.

5. Procédé selon l'une des revendications 1 à 4, **caractérisé en ce que** le mélange contenant les dérivés des alcools polyvalents contient seulement des quantités faibles d'eau, de préférence ≤ 5 % en poids d'eau, de manière mieux préférée ≤ 0,5 % d'eau.

6. Procédé selon l'une des revendications 1 à 5, **caractérisé en ce que** les temps de résidence par rapport à l'apport d'alcool vont de 0,1 à 20 heures, de préférence de 0,5 à 5 heures.

7. Procédé selon l'une des revendications 1 à 6, **caractérisé en ce que** l'on utilise un acide fort ou moyennement fort, de préférence l'acide phosphorique.

8. Procédé selon l'une des revendications 1 à 7, **caractérisé en ce que** les alcools polyvalents sont choisis dans le groupe constitué du triméthyloléthane, triméthylolpropane, triméthylolbutane, néopentylglycol et pentaérythrite, de manière préférée entre toutes du triméthylolpropane.

9. Procédé selon l'une des revendications 1 à 8, **caractérisé en ce que** l'on ajoute au mélange une amine secondaire avant l'addition d'acide.

10. Procédé selon l'une des revendications 1 à 9, **caractérisé en ce que** le procédé est réalisé pour des mélanges, dont l'alcool polyvalent n'a pas été encore éliminé par distillation.

11. Procédé selon l'une des revendications 1 à 9, **caractérisé en ce que** le procédé est réalisé pour des mélanges, dont l'alcool polyvalent a déjà été éliminé par distillation.
